(19) Europäisches Patentamt
European Patent Office
Office européen des brevets

(11) EP 1 110 552 A1

(12) EUROPEAN PATENT APPLICATION

(43) Date of publication:
27.06.2001 Bulletin 2001/26

(51) Int Cl.[7]: A61K 31/505, A61K 45/06, C07D 239/94, A61P 35/00

(21) Application number: 99403247.2

(22) Date of filing: 22.12.1999

(84) Designated Contracting States:
AT BE CH CY DE DK ES FI FR GB GR IE IT LI LU MC NL PT SE
Designated Extension States:
AL LT LV MK RO SI

(71) Applicant: Aventis Pharma S.A.
92160 Antony (FR)

(72) Inventors:
- Mignani, Serge
92290 Châtenay Malabry (FR)
- Debussche, Laurent
91200 Athis Mons (FR)
- Maratrat, Michel
94290 Villeneuve le Roi (FR)

(74) Representative: Le Pennec, Magali et al
RHONE-POULENC RORER SA,
Direction des Brevets,
20 Avenue Raymond Aron
92165 Antony Cédex (FR)

# (54) Use of a compound with affinity for the mitochondrial benzodiazepine receptor in cancer therapy

(57) The present invention relates in particular to a combination product comprising at least one compound with affinity for the mitochondrial benzodiazepine receptor, and to at least one apoptosis-inducing agent for simultaneous or separate use or for use spread out over time, which is intended for the treatment of cancer. Another aspect of the present invention relates to the use of the said compound and/or of the said combination product for the manufacture of a medicinal product intended to facilitate the induction of apoptosis.



Printed by Jouve, 75001 PARIS (FR)

## Description

The present invention relates in particular to a combination product comprising at least one compound with affinity for the mitochondrial benzodiazepine receptor, and to at least one apoptosis-inducing agent for simultaneous or separate use or for use spread out over time, which is intended for the treatment of cancer. Another aspect of the present invention relates to the use of the said compound adn/or of the said combination product for the manufacture of a medicinal product intended to facilitate the induction of apoptosis.

The methods currently used in the treatment of cancer are mainly radiotherapy and chemotherapy. These techniques consist in eradicating the tumor cells identified by means of localized irradiations or by means of pharmacological inducers of cell death. However, these therapeutic approaches are not specific to the tumor cells alone. Indeed, the neighboring tissues are also eradicated and vey high toxicity is observed. Considering that the natural programs of cell death (or apoptosis) and senescence no longer function in tumor cells, one approach for treating cancer might lie in re-establishing these programs.

In general, apoptosis is characterized by three phases : an initiation phase in which the various death stimuli take so-called « private » pathways to converge on a common effector phase, which leads finally to the degradation phase characterized by the characteristic biochemical symptoms of cell death. The effector phase is carrried out by the mitochondrial permeability transition pore, which is the true sensor of cell death since its open or closed conformations determine the fate of the cells. These different conformations can be induced by many ligands for the components of this permeability transition pore.

The mitochondrial permeability transition pore, commonly known as the mega-channel or multi-conductance channel, participates in regulating the lever of calcium in the matrix, the pH and the transmembrane potential ($\triangle\psi_m$) in the mitochondria. This pore (PT) thus functions as a channel which is dependent on $Ca^{2+}$, the voltage, the pH and the redox potential with several levels of conductance and little selectivity with respect to the ions (Zoratti et al. 1995, Kinnally et al. 1996, Bernardi et al. 1996, and Ichas et al. 1997). Recently, it has been demonstrated that opening of the PT pore, which is regulated by Bcl-2, is a critical event in the process leading to apoptosis (Kroemer et al. 1997a and 1997b). Opening of the PT pore allows dissipation of the mitochondrial internal transmembrane potential ($\triangle\psi_m$), the consequence of which is to disrupt the integrity of the outer membrane, leading to the release of mitochondrial intermembrane proteins (Zamzami et al. 1996, Susin et al. 1997a, Kantrow et al. 1997 and Ellerby et al. 1997). In point of fact, the release of intramembrane proteins, such as cytochrome c, and/or the dissipation of the $\triangle\psi_m$ are common elements of the early phase of apoptosis (Kroemer et al. 1997a and 1997b, Liu et al. 1996, Kluch et al. 1997, Yang 1997 and Susin et al. 1997b). Depending on the experimental system and the cell type, an increase in the volume of the matrix causes either a physical disruption of the outer mitochondrial membrane and then dissipation of the $\triangle\psi_m$ (Kluck et al. 1997, Yang et al. 1997 and Vander Heiden et al. 1997) or a disruption of the outer membrane and simultaneous dissipation of the $\triangle\psi_m$ of the inner membrane (Zamzami et al. 1996 and Susin et al. 1996a). Regarding the theoretical aspect, the authors mentioned above have postulated that the increase in the volume of the matrix, which precedes the reducation of the $\triangle\psi_m$ might be controlled by opening the PT pore. In this sense, the PT pore can operate both at a level of low and reversible conductance (which would give rise to an influx of ions and water into the mitochondrial matrix), and at a level of high and irreversible conductance (which would lead to disruption of the $\triangle\psi_m$).

The PT pore is a multiprotein complex formed at the site of contact between the inner and outer mitochondrial membranes. Co-localization of the PT pore and of the Bcl-2 oncoprotein is observed (De Jong et al. 1994). The exact molecular composition of this pore remains an enigma. However, it is known that proteins of the cytosol (hexokinase), of the outer membrane (mitochondrial benzodiazepine receptor [mBzR], mitochondrial porin, commonly referred to as the voltage-dependent anion channel), the intermembrane space (creatine kinase), the inner membrane (adenine nucleotide translocator, ANT) and the matrix (cyclophilin D) are involved in the formation of the PT pore and/or in its regulation (Zoratti et al. 1995, Beutner et al. 1996, McEnery 1992, Kinnally et al. 1993, O'Gorman 1997). The PT pore is regulated by multiple endogenous effectors, which is in accordance with its complex composite architecture. Among these effectors are the local ions and the pH gradient, ADP/ATP, NADPH and the molecules involved in transduction of the apoptotic signal, such as $Ca^{2+}$ or oxygen-reactive species. Thus, some of the subunits of the PT pore might constitute pharmacological targets for modulating apoptosis.

Compounds belonging to the isoquinolinecarboxamide family have been described in US 4,801,595 as being useful for the treatment of hypertension. In this family, PK11195 (1-(2-chlorophenyl)-N-methyl-N-(1-methylpropyl)-3 -isoquinoline-carboxamide) is known as being a prototypic antagonist ligand for the peripheral benzodiazepine receptor mBzR (Ripond et al. 1991 and Joseph-Liauzun et al. 1997). More particularly, WO 93/11771 relates to the use of molecules such as PK11195 for the treatment of diseases of the central nervous system, in particular trauma.

Certain compounds with affinity for the mitochondrial benzodiazepine receptor, especially the isoquinoline carboxamide PK11195 allow opening of the mitochondrial permeability pore (Hirsch et al., Exp. Cell Res. 1998, 241 (2), pp.426-34), facilitates the induction of apoptosis and reverses Bcl-2-mediated cytoprotection. The experiments which have led to the invention demonstrate that compounds of formula (I) are also ligand of the mitochondrial benzodiazepine receptor and exhibit the same activities.

Cancer therapy currently consists in using radiotherapy, chemotherapy or a combination of these. These two methods have harmful side effects which are very poorly tolerated by the patients. Thus, the use of pharmacological agents designed to increase the susceptibility of tumor cells to apoptosis induction is very advantageous, since it allows the use of lower doses of chemotherapy or radiotherapy products, thereby minimizing their side effects. Components with strong affinity for the PT pore subunits, mentioned above, are the subject of the present invention since they facilitate apoptosis and thus allow the use of lower doses of products with strong side effects. This also makes it possible to improve the efficacy of certain treatments.

Thus, no document of the prior art either discloses or suggests the present invention as described below.

Description of the invention

Thus, the present invention relates to a combination product comprising at least one compound of the invention, and to at least one apoptosis-inducing agent for simultaneous or separate use or for use spread out over time, which is intended for the treatment of cancer.

The term cancer is used in a broad sense which includes any neoplasia (for example cancers, sarcomas, lymphomas and leukemias).

The compounds of the invention are those represented by formula I. Their formulae and the processes for their preparation are described in EP 210,084 A1, which is incorporated by way of reference in the description.

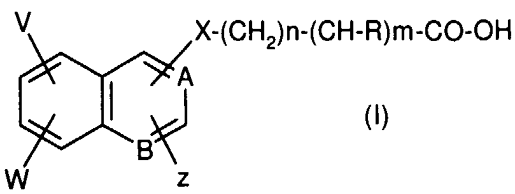

(I)

in which A represents a nitrogen atom or a CH group,

- B represents a nitrogen atom or a CH group,
- V and W, which may be identical or different, represent hydrogen atoms or halogen atoms (fluorine, chlorine or bromine), alkyl or alkoxy groups comprising 1 to 3 carbon atoms, or nitro or trifluoromethyl groups,
- Z is fixed in an ortho or para position relative to B and represents a phenyl, thienyl or pyridyl radical, or a phenyl radical substituted with one or two substituents taken from halogen atoms, alkyl and alkoxy groups comprising 1 to 4 carbon atoms, and trifluoromethyl or nitro groups,
- the chain $-X-(CH_2)_n-(CH-R)_m-CO-OH$ is fixed in an ortho or para position relative to B,
- R represents a hydrogen atom or an alkyl group comprising 1 to 3 carbon atoms,
- X represents a group $CH-R_3$, $N-R_4$, SO or $SO_2$ or an oxygen or sulfur atom,
- $R_3$ represents a hydrogen atom or an alkyl group comprising 1 to 3 carbon atoms,
- $R_4$ represents an alkyl group comprising 1 to 3 carbon atoms,
- m is equal to 0 or 1,
- n is equel to 0, 1 or 2.

In other words, the compounds of formule (I) correspond to one of the formulae (Ia) and (Ib)

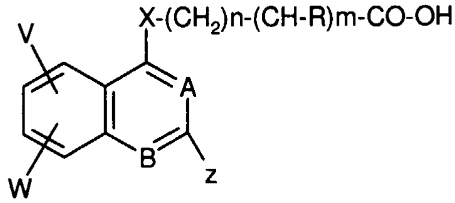

(Ia)

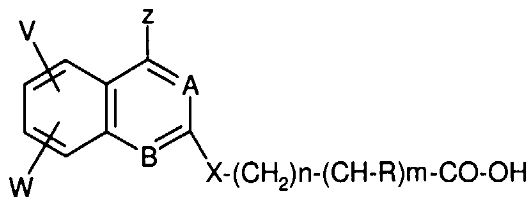

(Ib)

in which A, B, V, W, X, Z, R, n and m have the meanings given above.

When B is N and A is CH that is to say when formula (I) is a quinoline the process of preparation of the compounds of the following formula

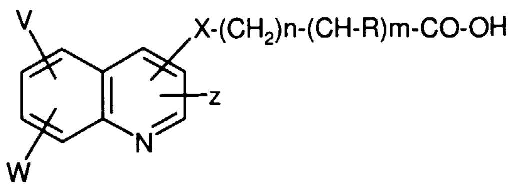

where X is O is described in EP 362,006.

Compounds of formula (Ic)

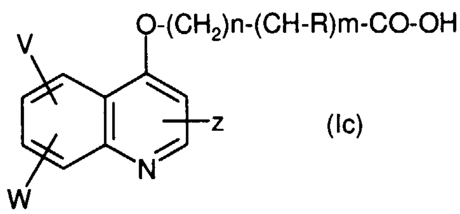

(Ic)

where V, W, Z, n and M have the same meaning as above are for example prepared by condensation of a quinolone of formula (II)

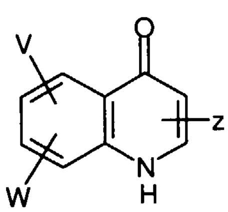

(II)

with a derivative of formula

$$Hal\text{-}(CH_2)_n\text{-}(CHR)_m\text{-}COOR' \qquad (III)$$

where Hal is an halogen chosen from chlorine or bromine and R' is a small alkyl group ($C_1$-$C_3$). This condensation is carried out in presence of alkali carbonate such as sodium or potassium in an organic solvent such as a ketone (2 butanone) at a temperature between 20°C and the boiling point of the solvent.

The acids are further obtained by hydrolysis of the obtained esters by mean of aqueous soda solution at boiling temperature.

The quinolones of formula (II) are preferably obtained by cyclisation of a compound of formula

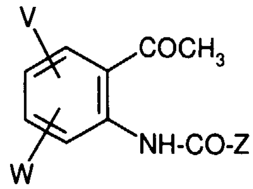

(IV)

in an aromatic solvent such as benzene or toluene in presence of sodium tertiobutylate.

The compounds of formula (IV) are preferably obtained by condensation of product of formula (V)

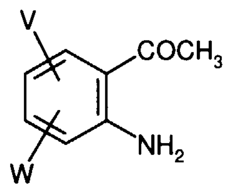

(V)

with a product of formula (VI)

Cl-CO-Z

in an aromatic solvent in presence of pyridine at room temperature.

The compounds of formula (V) are obtained by oxidation of a compound of formula (VII)

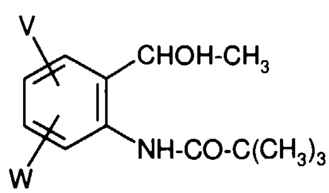

with for example chromic anhydride in a ketone solvent at a temperature situated between -5°C and 0°C, followed by reaction with an alcoholic acid to remove the amine protecting group.

Compounds of formula (VII) are prepared for example by action of acetaldehyde on a derivative of formula (VIII)

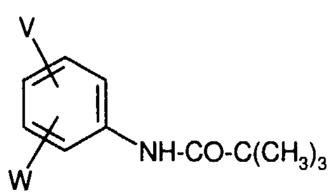

(VIII)

in presence of butyllithium in a unreactive solvent.

Compounds of formula (VIII) can be obtained by action of pivaloyl chloride on a derivative of formula

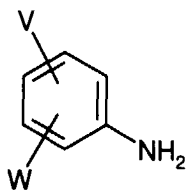

in an organic solvent such as benzene or toluene in presence of trialkylamino or pyridine.

When X is S instead of an alkylation a thionation is carried out in presence of $P_2S_5$ in xylene. The reaction is described in Ann. 574, 226, 1951 or with $H_2S$/HCl/Ether as it is described in Berichte, 93, 431, 1960.

When X is NH compound (II) is reacted with bromine as described in Heterocyclic Chemistry, vol. II, p. 393, 1965. The bromine compound is further aminated following the reaction described in Recueil Travaux Chimiques des Pays-Bas, vol. 93, p. 143, 1974 and further reacted with compound of formula (III).

Compounds of formula I in which A and B each represent a nitrogen atom, Z is bonded at the ortho position relative to B and the chain Alk-$CO_2H$ is bonded at the para position relative to B may be prepared by the action of a compound of formula II in which $R_1$ represents a hydrogen atom or a $C_1$-$C_4$ alkyl radical and $R_2$ represents hydrogen or a $C_1$-$C_6$ alkyl radical or a $C_1$-$C_2$ alkoxycarboxy group on a compound of formula III in which $R_3$ and $R_4$, which are identical or different, each represent hydrogen or a $C_1$-$C_4$ alkyl radical.

$$R_2R_1CH\text{-}CO_2alk \qquad (II)$$

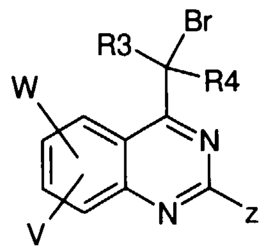

(III)

This reaction is preferably carried out in an inert solvent medium such as dimethylformamide, tetrahydrofurane or dimethylsulfoxide, in the presence of a base such as a tertiary amine (for example triethylamine) or an aromatic amine (for example pyridine), an inorganic base such as an alkali metal hydroxide (for example soda or potassium hydroxide) or in the presence of an alkali metal hydride such as sodium hydride or potassium hydride, at a temperature between 20°C and the boiling point of the reaction medium. The intermediate ester is hydrolysed to the corresponding acid by the action of an acid such as hydrochloric acid in aqueous solution at a temperature between 20°C and the boiling point of the reaction medium.

The derivatives of general formula II are commercially available or may be obtained by applying or adapting the methods described by C. Larock, «Comprehensive Organic Transformations», Ed. VCH, pages 863 and 913 (1989) and when $R_2$ represents a $C_1$-$C_2$ alkoxycarboxy group by, for example, condensation of a halide Hal-$R_1$ in which $R_1$ represents a $C_1$-$C_2$ alkyl radical on a di($C_1$-$C_2$)alkyl malonate. This reaction is preferably carried out in an inert solvent medium such as dimethylformamide, tetrahydrofurane or dimethylsulfoxide, in the presence of an alkali metal hydride such as sodium hydride or potassium hydride, at a temperature between 20°C and the boiling point of the reaction medium.

Compounds of formula III in which $R_3$ and $R_4$, are identical or different and each represents hydrogen or a $C_1$-$C_4$ alkyl radical may be prepared by the action of a corresponding compound of formula IV in which $R_3$ and $R_4$, which are identical or different, each represent hydrogen or a $C_1$-$C_4$ alkyl radical with a brominating agent such as bromine or N-bromosuccinimide, optionally in the presence of a peroxide such as benzoyl peroxide in an inert solvent such as carbon tetrachloride or an ether such as tetrahydrofurane at a temperature between 20°C and the boiling point of the reaction medium.

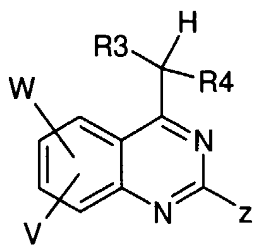

(IV)

Derivatives of general formula IV may be obtained by applying or adapting the methods described by Schofield, J. Chem. Soc., 1924, 1952; Kovendy, Chem. Ber. 98(4), 1049, 1965; Ikeda, Tetrahedron Lett., 27, 2347, 1976; Strekowski, 28(34), 4265, 1988 and Ried, Justus Liebigs Ann. Chem., 2007, 1976; Tamura, Chem. Pharm. Bull. 26(9), 2866, 1978; A.J. Boulton et A. McKillop, «Comprehensive Heterocyclic Chemistry», Volume 3, part 2B, pages 53 to 155.

The pure diastereoisomers of the compounds of formula (I ) can be isolated from the mixture by conventional methods such as chromatography, fractional crystallisation, salt formation and regeneration of acid.

The pure enantiomers of the compounds of formula (I) containing one or two asymmetric carbon atoms can be obtained by resolving the racemates, for exemple by chromatography on a chiral column according to W. H. PIRKLE et al, Asymmetric synthesis, vol. 1, Academic Press (1983) or alternatively by synthesis from chiral precursors.

The compounds of formula (I) where A and B are each N that is to say the quinazoline compounds are new compounds which are claimed as such.

The new compounds of formula are also claimed

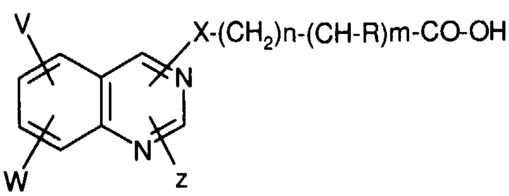

in which V and W, which may be identical or different, represent hydrogen atoms or halogen atoms (fluorine, chlorine or bromine), alkyl or alkoxy groups comprising 1 to 3 carbon atoms, or nitro or trifluoromethyl groups,

Z is fixed in an ortho or para position relative to N and represents a phenyl, thienyl or pyridyl radical, or a phenyl radical substituted with one or two substituents taken from halogen atoms, alkyl and alkoxy groups comprising 1 to 4 carbon atoms, and trifluoromethyl or nitro groups,
the chain $-X-(CH_2)_n-(CH-R)_m-CO-OH$ is fixed in an ortho or para position relative to N,
R represents a hydrogen atom or an alkyl group comprising 1 to 3 carbon atoms,
X represents a group $CH-R_3$, $N-R_4$, SO or $SO_2$ or an oxygen or sulfur atom,
R3 represents a hydrogen atom or an alkyl group comprising 1 to 3 carbon atoms,
$R_4$ represents an alkyl group comprising 1 to 3 carbon atoms,
m is equal to 0 or 1,
n is equel to 0, 1 or 2

with the proviso of *N*-[2-(4-chlorophenyl)-6, 7-dimethoxy-4-quinozolyl] glycin, *N*-[6-chloro-2-(2-thienyl)-4-quinazolyl] glycin and *N*-[6-bromo-2-(2-thienyl)-4-quinazolyl] glycin.

The salts, enantiomers and diastereoisomers are also included in the present invention.

The expression « apoptosis-inducing agent » denotes any substance which directly or indirectly affects the viability of a cell.

The said apoptosis-inducing agent of the present invention can be selected in particular from agents which damage DNA, glucocorticoid receptor ligands or pro-apoptotic second messengers.

These agents can also be selected from those commonly used in the treatment of cancer. Thus, the said pro-apoptotic second messenger is selected from glucocorticoid derivatives, from alkylating agents such as nitrogen mustards, for example cyclophosphamide, platinum complexes, for example cisplatin, ethyleneimine derivatives, dimethane sulfonoxyalkane derivatives or piperazine derivatives, from topoisomerase inhibitors such as topoisomerase-2 inhibitors, for example anthracyclines, epipodiphyllotoxins such as etoposide, topoisomerase-1 inhibitors, for example camptothecin derivatives, from antimetabolites such as antifolates, for example methotrexate, antipurines, for example 6-mercaptopurine, antipyrimidines, for example 5-fluorouracil, from antimitotic agents such as vinca alkaloids, taxoids such as taxol, taxotere, and from various cytolytic agents such as bleomycin, dacarbazine, hydroxycarbamide, asparaginase, mitoguazone and plicamycin.

These antineoplastic agents are described in Actualité Pharmaceutique No. 302 (Oct. 1992), pages 38 to 39 and 41 to 43, which are incorporated in the description by reference.

Preferably, the said apoptosis-inducing agent is chosen from gamma radiations, etoposide, doxorubicin, dexamethasone, ceramide such as ceramide C8, and lonidamine.

Some of the said anticancer agents are described more particularly in US 5,260,327 which relates to the use of lonidamine for treating metastases, in Official Gazette5,017,353 which relates to the use of lonidamine in combination with other anticancer agents, and in EP 291,151, which describes the use of phlorizine derivatives. These documents are incorporated into the description by reference.

The product according to the present invention can also contain a viral vector which contains a gene which codes for an enzyme for activating the abovementioned compounds and/or agents, for example thymidine kinase. Many patents relating to the use of activated suicide genes in specific tissues are found in the family of EP 415,731. Among these documents, which are incorporated into the description by reference, are : EP 494,776, EP 690,129, EP 657,540 and EP 657,541 which relate in particular to the manufacture of a medicinal product comprising a vector which contains a gene capable of catalyzing the conversion of a prodrug into the active substance. More particularly, EP 657,539 relates to the use of a thymidine kinase gene with cell specificity for the treatment of cancer.

Similarly, the product of the present invention can also comprise one or more pharmaceutically acceptable vehicles.

In another aspect, the present invention relates to the use of the product described above for the manufacture of a medicinal product intended for the treatment of cancer. In particular, the said medicinal product is intended to induce thedeath of tumor cells and/or facilitate apoptosis.

Bcl-2 is the prototypic representative of the family of apoptosis-inhibiting oncogenes which contributes both to the generation of cancer and which is responsible for the difficultiers in eradicating tumors. Most of the cytoprotective effects of Bcl-2 can be attributed to its capacities to protect the integrity of the mitochondrial membranes (Boise et al. 1997, Reed et al. 1997). It has been shown that Bcl-2 stabilizes mitochondrial membranes in various models of apoptosis (Zamzami et al. 1995, and Decaudain et al. 1997). However, it appears that Bcl-2 does not manage to inhibit apoptosis in certain cases, in particular apoptosis induced by diamide and by activation of caspase (Yasuhara et al. 1997, Strasser et al. 1995 and Huang et al. 1997). The effect of Bcl-2 is also overcome by treatment with a taxoid, such as paclitaxel (taxol), this agents allowing the hyperphosphorylation of Bcl-2 (Haldar et al. 1995 and 1996), and which promotes opening of the PT pore (Evtodienki et al. 1996).

Thus, the present invention proposes an alternative for overcoming the chemo- or radioresistance observed by mediation by Bcl-2. The treatment of cells with a ligand for mBzR makes cytoprotection with Bcl-2 largely obsolete. There is virtual stoechiometry between the expression of Bcl-2 and that of mBzR, at least as regards the lymphoid cell lines (Carayon et al. 1996). In addition, Bcl-2 protects isolated mitochondria against opening of the PT pore induced by low doses of protoporphyrin IX, which is an mBzR ligand, and this inhibition is suppressed by strong doses of protoporphyrin IX (Marchetti et al. 1996a). This suggests that a functional interaction exists between Bcl-2 and mBzR. On the other hand, the protection against opening of the PT pore, controlled by Bcl-2, is not overcome by increasing doses of the other target agents for the PT pore such as atractyloside, which is a ligand for the adenine translocator. Since the binding of mBzR does not bring about any negative regulation of the expression of Bcl-2 (Carayon et al. 1996), it thus seems probable that the conformational changes arising from the binding of PK11195 in the complex composed of mBzR and the PT pore indirectly affects the stability of the mitochondrial membrane and thus overcomes the anti-apoptotic function of Bcl-2. Consequently, the binding of a compound with affinity for the mitochondrial benzodiazepine receptor, such as compounds of the invention, represents an advantageous strategy in particular for overcoming resistance to chemotherapy and to radiotherapy.

The present invention will be further exemplified.

**Example 1 - Synthesie**

2-Methyl-3-(2-phenyl-4-quinazolinyl)propionic acid (dextrorotatory isomer)

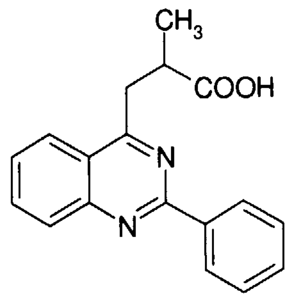

A solution of 19 g of N-[(2R)-2-(1-hydroxy-2-phenyl)ethyl]-2-methyl-3-(2-phenylquinazolin-4-yl)propionamide (isomer A) in 90 $cm^3$ of aqueous 37 % hydrochloric acid and 90 $cm^3$ of pure acetic acid is heated with stirring at a temperature in the region of the reflux temperature, under an inert atmosphere for 1 h 30 min. The mixture is then concentrated under reduced pressure (5 kPa) at a temperature in the region of 40°C, the residue is taken up in 600 $cm^3$ of water and brought to pH 10 with a sufficient amount of 28 % aqueous ammonia. The mixture is extracted with 100 $cm^3$ of ethyl ether. The alkaline phase is separated out after settling has taken place and brought to pH 5-6 with a sufficient amount of pure acetic acid. An oil precipitates and then crystallizes. After stirring for 16 hours at a temperature in the region of 20°C, the crystalline suspension is spin-dried and washed with water. The crystals are dried under vacuum (2 kPa) over phosphorus pentoxide at a temperature in the region of 40°C, to constant weight. 13.5 g of a white powder are obtained, 5 g of which are recrystallized from 50 $cm^3$ of ethanol. After spin-drying the crystals, they are dried to constant weight under reduced pressure (13 kPa) at a temperature in the region of 40°C. 4.5 g of 2-methyl-3-(2-phenyl-4-quinazolinyl)propionic acid are obtained in the form of a white solid melting at 180°C.

($\alpha_D^{20}$ = + 4.3 +/- 0.7 in 0.6% acetic acid)

*N*-[(2R)-2-(1-hydroxy-2-phenyl)ethyl]-2-methyl-3-(2-phenylquinazolin-4-yl)propionamide (isomer A)

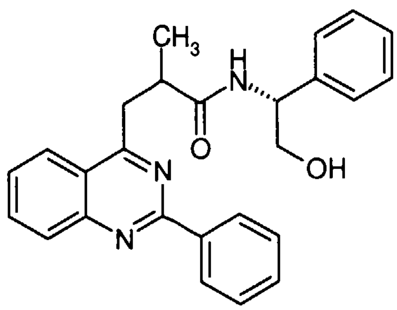

59.1 g of (2R,S)-2-methyl-3-(2-phenylquinazolin-4-yl)propionic acid, 1800 $cm^3$ of dichloromethane, 44.5 g of dipyridyl disulphide and 28 g of (R)-(-)-2-phenylglycinol are mixed together, in order, under an inert atmosphere with stirring. The solution obtained is cooled to about 5°C, followed by portionwise addition of 53 g of triphenylphosphine. The mixture is allowed to return to a temperature in the region of 20°C. After 21 hours, the reaction mixture is concentrated under reduced pressure (5 kPa) at a temperature in the region of 40°C and the residue is then taken up in 1800 $cm^3$ of ethyl acetate. The organic solution is washed with aqueous IN sodium hydroxide (1 x 450, 1 x 250, 1 x 100 $cm^3$) and then twice with 100 $cm^3$ of water. A precipitate forms, which is removed by filtration. The solution is then washed with 200 $cm^3$ of aqueous 10 % sodium dithionite solution, and then with 100 $cm^3$ of water and 100 $cm^3$ of saturated aqueous sodium chloride solution. After drying over magnesium sulphate and then concentrating under reduced pressure (5 kPa) at a temperature in the region of 40°C, 139 g of a brown lacquer are obtained. 46.5 g (i.e. 1/3 of the total mass) are purified by chromatography under an argon pressure of 1000 kPa on a column of silica gel (diameter 8 cm; mass of silica 1500 g), eluting at a rate of 30 $cm^3$/min. After collecting 2500 $cm^3$, 250 $cm^3$ fractions are collected. Fractions 9 to 13 are combined and then evaporated under reduced pressure (5 kPa) at a temperature in the region of 40°C. 7.8 g of a product are obtained, which product is recrystallized from 230 $cm^3$ of acetonitrile. 4.5 g of *N*-[(2R)-2-(1-hydroxy-2-phenyl)ethyl]-2-methyl-3-(2-phenylquinazolin-4-yl)propionamide (isomer A) are collected in the form of a white powder melting at 199°C.

(2R,S)-2-Methyl-3-(2-phenylquinazolin-4-yl)propionic acid

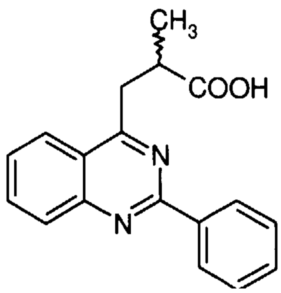

8 g of 60% sodium hydride are added portionwise to 125 $cm^3$ of anhydrous tetrahydrofuran under inert atmosphere and with stirring, followed by dropwise addition, at a temperature in the region of 20°C, of 34.8 g of diethyl methylmalonate dissolved in 125 $cm^3$ of tetrahydrofuran. 29.9 g of 4-bromomethyl-2-phenylquinazoline dissolved in 125 $cm^3$ of tetrahydrofuran are added to the solution obtained, at a temperature in the region of 20°C with stirring. The mixture is stirred for 1 hour at room temperature. After cooling to a temperature in the region of 10°C, the reaction mass is brought to pH 4 by addition of 10 $cm^3$ of pure acetic acid and is then diluted with 150 $cm^3$ of water. The solution obtained is partially evaporated under reduced pressure (5 kPa) at a temperature in the region of 40°C. The residue is taken up, with stirring, with 150 $cm^3$ of ethyl acetate and 100 $cm^3$ of water. The aqueous phase is separated out after settling has taken place and is then extracted twice more with 100 $cm^3$ of ethyl acetate. The combined organic extracts are washed with twice 50 $cm^3$ of water and then dried over magnesium sulphate, filtered and evaporated under reduced pressure (5 kPa) at a temperature in the region of 40°C. 49 g of an oil are obtained, which product is stirred for 4 hours with 250 $cm^3$ of aqueous 37% hydrochloric acid and 250 $cm^3$ of pure acetic acid at a temperature in the region of the boiling point. The reaction mass is evaporated under reduced pressure (5 kPa) at a temperature in the region of 50°C. The residue obtained is taken up in 600 $cm^3$ of water and then basified to pH 10, with stirring, at a temperature in the region of 10°C, with 125 $cm^3$ of potassium hydroxide. After addition of 200 $cm^3$ of diethyl ether and separation of the aqueous phase by settling, this aqueous phase is washed with twice 100 $cm^3$ of diethyl ether. The aqueous phase is stirred for 15 minutes with 2 g of vegetable charcoal and filtered through Celite. The filtrate is brought to pH 4 with a sufficient amount of pure acetic acid. The precipitate formed is spin-dried, washed with water and dried under vacuum (2 kPa) over phosphorus pentoxide, at a temperature in the region of 50°C. 18.5 g of (2R,S)-2-methyl-3-(2-phenylquinazolin-4-yl)propionic acid are obtained in the form of a beige-coloured solid. ($R^f$ = 0.63 in ethyl acetate, on Merck $60F_{254}$ silica plate).

4-Bromomethyl-2-phenylquinazoline

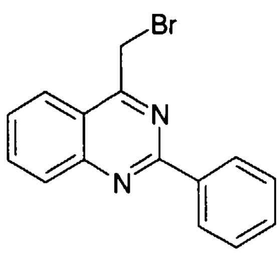

7.2 g of benzoyl peroxide are added with stirring, at a temperature in the region of 20°C, to a solution of 71.2 g of 4-methyl-2-phenylquinazoline in 660 $cm^3$ of carbon tetrachloride, followed by portionwise addition of 59 g of N-bromosuccinimide. The suspension obtained is brought to a temperature in the region of the boiling point for 2 h 30 min. After cooling the reaction mass to a temperature in the region of 20°C, the precipitate is spin-dried and washed with carbon tetrachloride. The filtrate is concentrated under reduced pressure (5 kPa) at a temperature in the region of 40°C. 108.7 g of an oil are obtained, which product is dissolved in 300 $cm^3$ of cyclohexane with heating. After cooling the solution to a temperature in the region of 20°C for 2 hours, the resultant crystalline mass is spin-dried, washed with 200 $cm^3$ of a cyclohexane/petroleum ether (30-60°C) mixture (50/50 by volume) and then washed with 3 times 200 $cm^3$ of petroleum ether (30-60°C). The crystals are dried under reduced pressure (2 kPa) at a temperature in the region of 40°C, to constant weight. 67.8 g of 4-bromomethyl-2-phenylquinazoline are obtained in the form of a pale yellow solid. ($R^f$ = 0.3 in an 80/15/5 by volume cyclohexane/dichloromethane/ethyl acetate mixture, on Merck $60F_{254}$ silica plate).

4-Methyl-2-phenylquinazoline can be prepared according to Alexandre Kovendi and Magda Kircz, Chem. Ber. 98 (4), 1049-59, 1965.

**Example 2 - Biological activity.**

The biological activity of the compounds according to the present invention can be illustrated as follows.

**A. Assay for apoptosis induction in a cell population**

To quantify in a cell population the number of apoptotic cells according to their DNA content, an assay method which is based on FACS analysis (fluorescence activated cell sorting) and which uses cell fluorescence labelling with two intercalating agents has been adapted from the method described by Pollack A. and Ciancio G. (Pollack A et Ciancio G.In: Flow Cytometry, Darzynkiewicz Z, Crissman HA (eds). Academic Press, San Diego, CA, 1991, pp 19-24). After trypsinisation, cells were rinsed with PBS (« Phosphate Buffer Saline purchased from, for example, Gibco BRL) and fixed in 1 ml cold ethanol during 30 minutes. Cells were then rinsed twice in 2 ml PBScontaining 0,5 % Tween 20, then resuspended in 1 ml coloration medium (PBS-Tween 20 0,5 % containing 1 mg/ml boiled Rnase, 10 µg/ml Propidium iodide and 4 µg/ml DAPI-4',6-diamidino-2-phenylindole dihydrochloride).FACS analysis was run after UV excitation. Studies were run with cell population grown in Nunc 6-well plates seeded with 100 000 cells by well in 2 ml complete medium, various compounds inducing or facilitating apoptose being incorporated 36 hours after seeding and FACS analysis being carried out between 24 and 48 h after adding the compound(s).

**B. Assay method for inhibition of Bcl2-dependent anti-apoptotic activity in a cell line that conditionnally overexpresses Bcl-2.**

By means of stable transfection techniques and by using selection agents such as neomycin, hygromycin, zeocin or puromycin, clones derived for instance from NCI-H1299 cell line (obtained from ATCC) can be established in order to have a conditional expression of Bcl2 strictly depending on tetracyclin or anhydrotetracyclin concentration in the culture medium. To do that, one can use the technique described by Gossen and Bujard (Gossen M & Bujard H. (1992). *Proc Natl Acad Sci U S A*, 89, 5547-51; Nucleic Acids Res 1993 Sep 11;21(18):4411-2), with the possibility to adapt it while keeping the features of the transcriptional regulation system described by this authors. We have been able to establish clones that overexpress Bcl2 protein in absence of tetracyclin or anhydrotetracyclin in culture medium, whereas in presence of 0.5 mg/1 anhydrotetracyclin, the protein was not detectable by western blotting by using anti-Bcl2 antibody, clone 124 purchased from DAKO and by using a protocol described by Venot et al. (Venot, C., Maratrat, M., Dureuil, C., Conseiller, E., Bracco, L., and Debussche, L. 1998. EMBO J. 17:4668-4679).

By using above-mentioned procedure, we have been able to characterize clones which exhibit, in absence of tetracyclin in culture medium, significantly lower sensitivity to apoptosis induction by a range of concentrations of apoptosis inducing agents than in presence of tetracyclin, which means when they overexpress Bcl-2 protein rather than they do not. Apoptosis inducing agents tested have been for example adriamycin (10-100 µg/ml), terbutyl-hydroperoxide (25-100 µM), vincristine (0.03-0.003 µg/ml) and camptothecin (0.01-0.1 µg/ml). To quantify apoptosis induction, one can for example use the assay method described in A.

One possible way to assay inhibition of anti-apoptotic activity in a conditionally Bcl-2 overexpressing cell line, this inhibition being due to treatment which can be for example incorporation of a chemical compound, can be to quantify, by using assay method of example 1, the percentage of apoptosis induction in a cell population coming from, for example, one of the above-pmentioned clones, in the 4 following situations:

1) the chemical compound to be evaluated is incorporated in culture medium with an apoptosis inducing agent such as for instance one of the above-mentioned agents and the clone is grown in absence of tetracyclin or anhydrotetracyclin, which means when it overexpress Bcl-2. In this situation, the percentage of apoptotic cells P1 is determined.
2) The same apoptosis inducing agent as the one used in situation I is incorporated alone in the culture medium and the clone is grown in absence of tetracyclin or anhydrotetracyclin, which means when it overexpress Bcl-2. In this situation, the percentage of apoptotic cells P2 is determined.
3) the chemical compound to be evaluated is incorporated in culture medium with the same apoptosis inducing agent as the one used in the situation 1 and the clone is grown in presence of tetracyclin (1 µg/ml) or anhydrotetracyclin(0.5 µg/ml), which means when it does not overexpress Bcl-2. In this situation, the percentage of apoptotic cells P3 is determined.
4) The chemical compound is incorporated alone in the culture medium and the clone is grown in presence of tetracyclin (1 µg/ml) or anhydrotetracyclin(0.5 µg/ml), which means when it does not overexpress Bcl-2. In this situation, the percentage of apoptotic cells P4 is determined.

One can calculte ratio R = 100 x (P1-P2)/(P3-P4). When R=100, inhibition of anti-apoptotic activity due to

Bcl-2 overexpression is 100 % at the concentration of the chemical compound tested. When R= 0, the compound does not inhibit this activity at the tested concentration. A series of results can be obtained from a range of concentrations of tested compound. One can calculate from these results an IC50.

**C. compounds of the invention facilitate induction of cell death or apoptosis in tumor cell lines**

The tumor cell lines that can be used, for example, to monitor activity of compounds that facilitate induction of cell death or apoptosis can be MDA-MB-231, MDA-MB-468, DU-145, PC-3 and HepG2.
Cells derived from eg. one of the above-mentionned tumor cell lines are incubated with 1 to 50 μM concentration of one compound of the invention and a cell-death or apoptosis inducing agent. Cell death or apoptosis inducing agents tested can be for example taxol (0.0001-0.01 μg/ml), taxotere (0.0001-0.01 μg/ml), adriamycin (1-100 μg/ml), terbutylhydroperoxide (25-500 μM), vincristine (0.03-10 μg/ml), camptothecin (0.01-0.1 μg/ml) or appropriate radiation dosage. To quantify apoptosis induction, one can for example use the assay method described in A. The typical activity to be observed with the compound of the invention is a significant induction of cell-death or apoptosis by concentrations of a cell-death or apoptosis-inducing agent lower than the concentrations required to induce the same extent of cell death or apoptosis induction in the same experimental conditions without one of the compounds of the invention.

**D. Compounds of the invention bind to the mitochondrial/peripheral benzodiazepine receptor.**

It is established that PK11195 is a specific nanomolar range ligand of the mitochondrial/peripheral benzodiazepine receptor (see introduction). Specific binding of compound of the invention to the mitochondrial/peripheral benzodiazepine receptor can be determined by radioligand binding assays similar to the ones described in Hardwick M. et al. (Hardwick M. et al, Cancer Research, 1999, 59, pp 831-842). Briefly, the principle of such assays is to measure displacement activity for cellular or protein binding of nmolar concentrations of radiolabelled PK11195 by nmolar to molar concentration of the compound of the invention and then to determine IC50 for displacement activity. Compounds of the invention have been shown to demonstrate, by such assay methods, IC50 in the submicromolar range.

## Claims

**1.** Combination product comprising at least one compound of formula (I) and at least one apoptosis-inducing agent for simultaneous or separate use or for use spread out over time, which is intended for the treatment of cancer, characterized in that the compound of formula (I) has the following formula

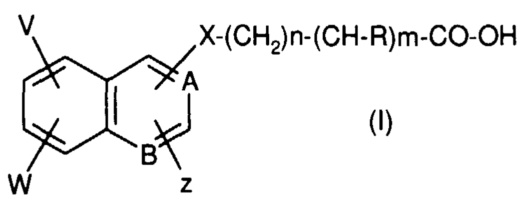

(I)

in which

A represents a nitrogen atom or a CH group,

B represents a nitrogen atom or a CH group,

V and W, which may be identical or different, represent hydrogen atoms or halogen atoms (fluorine, chlorine or bromine), alkyl or alkoxy groups comprising 1 to 3 carbon atoms, or nitro or trifluoromethyl groups,

Z is fixed in an ortho or para position relative to B and represents a phenyl, thienyl or pyridyl radical, or a phenyl radical substituted with one or two substituents taken from halogen atoms, alkyl and alkoxy groups comprising 1 to 4 carbon atoms, and trifluoromethyl or nitro groups,

the chain $-X-(CH_2)_n-(CH-R)_m-CO-OH$ is fixed in an ortho or para position relative to B,

R represents a hydrogen atom or an alkyl group comprising 1 to 3 carbon atoms,

X represents a group CH-$R_3$, N-$R_4$, SO or $SO_2$ or an oxygen or sulfur atom,

$R_3$ represents a hydrogen atom or an alkyl group comprising 1 to 3 carbon atoms,

$R_4$ represents an alkyl group comprising 1 to 3 carbon atoms,

m is equal to 0 or 1,

n is equal to 0, 1 or 2.

**2.** Combination according to claim 1 in which compounds of formula (I) correspond to one of the formulae (Ia) and (Ib)

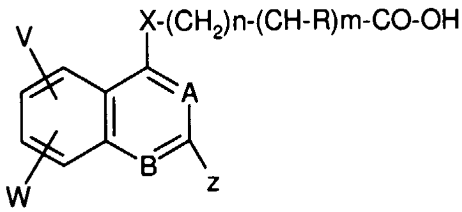

(Ia)

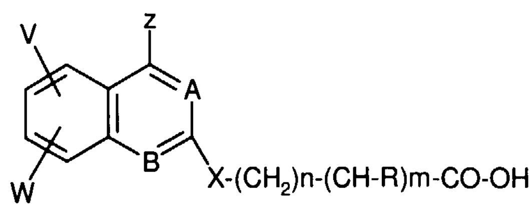

(Ib)

in which A, B, V, W, X, Z, R, n and m have the meanings given above.

**4.** Product according to one of claims 1 to 3, characterized in that the said apoptosis-inducing agent is selected from agents which damage DNA, natural or synthetic ligands of the glucocorticoid receptor, or pro-apoptotic second messengers.

**5.** Product according to claim 4, characterized in that the said agent is preferably selected from γ-radiations, etoposide, doxorubicin, dexamethasone, ceramide such as ceramide C8, and lonidamine.

**6.** Product according to claim 4, characterized in that the said pro-apoptotic second messenger is selected from glucocorticoid derivatives, from alkylating agents such as nitrogen mustards, for example cyclophosphamide, platinum complexes, for example cisplatin, ethyleneimine derivatives, dimethane sulfonoxyalkane derivatives, or piperazine derivatives, from topoisomerase inhibitors such as topoisomerase-2 inhibitors, for example anthracyclines, epipodophyllotoxin such as etoposide, topoisomerase-1 inhibitoirs, for example camptothecin derivatives, from antimetabolites such as antifolates, for example methotrexate, antipurines, for example 6-mercaptopurine, antipyrimidines, for example 5-fluorouracil, from antimitotic agents such as vinca alkaloids, taxoids such as taxol, taxotere, and from various cytolytic agents such as bleomycin, dacarbazine, hydroxycarbamide, asparaginase, mitoguazone and plicamycin.

**7.** Product according to one of claims 1 to 6, characterized in that it also comprises a viral vector which contains a gene which codes for thymidine kinase.

**8.** Product according to one of claims 1 to 7, characterized in that it also comprises one or more pharmaceutically acceptable vehicles.

**9.** Use of product according to claim 8 for the manufacture of a medicinal product intended for the treatment of cancer.

**10.** Use of a product according to claim 8 for the manufacture of a medicinal product intended to induce the deathn of tumor cells. 11 - Products of formula

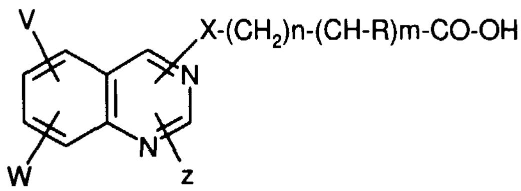

in which V and W, which may be identical or different, represent hydrogen atoms or halogen atoms (fluorine, chlorine or bromine), alkyl or alkoxy groups comprising 1 to 3 carbon atoms, or nitro or trifluoromethyl groups,

Z is fixed in an ortho or para position relative to N and represents a phenyl, thienyl or pyridyl radical, or a phenyl radical substituted with one or two substituents taken from halogen atoms, alkyl and alkoxy groups comprising 1 to 4 carbon atoms, and trifluoromethyl or nitro groups,
the chain $-X-(CH_2)_n-(CH-R)_m-CO-OH$ is fixed in an ortho or para position relative to N,
R represents a hydrogen atom or an alkyl group comprising 1 to 3 carbon atoms,
X represents a group $CH-R_3$, $N-R_4$, SO or $SO_2$ or an oxygen or sulfur atom,
$R_3$ represents a hydrogen atom or an alkyl group comprising 1 to 3 carbon atoms,
$R_4$ represents an alkyl group comprising 1 to 3 carbon atoms,
m is equal to 0 or 1,
n is equel to 0, 1 or 2

with the proviso of N-[2-(4 chlorophenyl)-6, 7-dimethoxy-4-quinozolyl] glycin, N-[6-chloro-2-(2 thienyl)-4-quinazolyl] glycin and N-[6-bromo-2-(2 thienyl)-4-quinazolyl] glycin.

European Patent Office

# EUROPEAN SEARCH REPORT

Application Number
EP 99 40 3247

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | DE 197 56 388 A (HOECHST MARION ROUSSEL DE GMBH) 24 June 1999 (1999-06-24)<br>* claims 1-7 *<br>* example 34 *<br>--- | 1-11 | A61K31/505<br>A61K45/06<br>C07D239/94<br>A61P35/00 |
| A,D | EP 0 210 084 A (RHONE-POULENC SANTE) 28 January 1987 (1987-01-28)<br>* claims 1-11 *<br>* page 77, line 18 - line 21 *<br>--- | 1-11 | |
| A,D | EP 0 362 006 A (RHONE-POULENC SANTE) 4 April 1990 (1990-04-04)<br>* claims 1-7 *<br>* page 12, line 20 - line 21 *<br>--- | 1-11 | |
| A,D | HIRSCH T ET AL: "PK11195, a ligand of the mitochondrial benzodiazepine receptor, facilitates the induction of apoptosis and reverses Bcl-2-mediated cytoprotection"<br>EXPERIMENTAL CELL RESEARCH,US,SAN DIEGO, CA,<br>vol. 241, 15 June 1998 (1998-06-15), pages 426-434, XP002098088<br>ISSN: 0014-4827<br>* the whole document *<br>---<br>-/-- | 1-10 | TECHNICAL FIELDS SEARCHED (Int.Cl.7)<br>A61K<br>C07D |

The present search report has been drawn up for all claims

| Place of search | Date of completion of the search | Examiner |
|---|---|---|
| BERLIN | 12 May 2000 | Siatou, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 99 40 3247

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | HARDWICK M ET AL: "Peripheral - type benzodiazepine receptor (PBR) in human breast cancer: correlation of breast cancer cell aggressive phenotype with PBR expression, nuclear localization, and PBR-mediated cell proliferation and nuclear transport of cholesterol" CANCER RESEARCH,US,AMERICAN ASSOCIATION FOR CANCER RESEARCH, BALTIMORE, MD, vol. 59, no. 4, 15 February 1999 (1999-02-15), pages 831-842, XP002114583 ISSN: 0008-5472 * abstract * ----- | 1-10 | |
| | | | TECHNICAL FIELDS SEARCHED (Int.Cl.7) |

The present search report has been drawn up for all claims

| Place of search | Date of completion of the search | Examiner |
|---|---|---|
| BERLIN | 12 May 2000 | Siatou, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 99 40 3247

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2000

| Patent document cited in search report | Publication date | Patent family member(s) | Publication date |
|---|---|---|---|
| DE 19756388 A | 24-06-1999 | AU 2270899 A | 12-07-1999 |
| | | WO 9932460 A | 01-07-1999 |
| EP 210084 A | 28-01-1987 | FR 2582514 A | 05-12-1986 |
| | | AT 40689 T | 15-02-1989 |
| | | AT 47840 T | 15-11-1989 |
| | | AU 579472 B | 24-11-1988 |
| | | AU 5803086 A | 04-12-1986 |
| | | AU 579473 B | 24-11-1988 |
| | | AU 5803186 A | 04-12-1986 |
| | | CA 1264160 A | 02-01-1990 |
| | | CA 1251206 A | 14-03-1989 |
| | | DE 3662045 D | 16-03-1989 |
| | | DE 3666813 D | 14-12-1989 |
| | | DK 252286 A | 01-12-1986 |
| | | DK 252386 A | 01-12-1986 |
| | | EP 0205375 A | 17-12-1986 |
| | | ES 555557 D | 16-04-1987 |
| | | ES 8704731 A | 01-07-1987 |
| | | ES 555558 D | 16-04-1987 |
| | | ES 8704732 A | 01-07-1987 |
| | | ES 557166 D | 01-05-1987 |
| | | ES 8705225 A | 16-07-1987 |
| | | ES 557167 D | 16-11-1987 |
| | | ES 8800660 A | 01-02-1988 |
| | | ES 557168 D | 01-05-1987 |
| | | ES 8705226 A | 16-07-1987 |
| | | ES 557169 D | 01-05-1987 |
| | | ES 8705227 A | 16-07-1987 |
| | | ES 557170 D | 01-05-1987 |
| | | ES 8705228 A | 16-07-1987 |
| | | GR 861368 A | 29-09-1986 |
| | | GR 861369 A | 29-09-1986 |
| | | HU 42071 A,B | 29-06-1987 |
| | | HU 42072 A,B | 29-06-1987 |
| | | IL 78968 A | 31-07-1989 |
| | | IL 78969 A | 31-07-1989 |
| | | JP 62005946 A | 12-01-1987 |
| | | JP 62000064 A | 06-01-1987 |
| | | NO 862133 A | 01-12-1986 |
| | | NO 862134 A | 01-12-1986 |
| | | NZ 216330 A | 26-04-1989 |
| | | NZ 216331 A | 26-04-1989 |
| | | PT 82673 A,B | 01-06-1986 |
| | | PT 82674 A,B | 01-06-1986 |
| | | SU 1440342 A | 23-11-1988 |

EPO FORM P0459

For more details about this annex : see Official Journal of the European Patent Office, No. 12/82

**ANNEX TO THE EUROPEAN SEARCH REPORT ON EUROPEAN PATENT APPLICATION NO.** EP 99 40 3247

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2000

| Patent document cited in search report | Publication date | Patent family member(s) | Publication date |
|---|---|---|---|
| EP 210084 A | | SU 1508957 A | 15-09-1989 |
| | | SU 1470183 A | 30-03-1989 |
| | | SU 1470182 A | 30-03-1989 |
| | | SU 1544186 A | 15-02-1990 |
| | | SU 1537135 A | 15-01-1990 |
| | | SU 1614759 A | 15-12-1990 |
| | | US 4788199 A | 29-11-1988 |
| EP 362006 A | 04-04-1990 | FR 2636327 A | 16-03-1990 |
| | | AU 4128189 A | 05-04-1990 |
| | | DK 449989 A | 14-03-1990 |
| | | HU 55390 A | 28-05-1991 |
| | | JP 2115169 A | 27-04-1990 |
| | | NO 893653 A | 14-03-1990 |
| | | NZ 230615 A | 25-06-1991 |
| | | PT 91712 A | 30-03-1990 |
| | | SU 1709911 A | 30-01-1992 |
| | | US 5017576 A | 21-05-1991 |
| | | ZA 8906948 A | 27-06-1990 |

EPO FORM P0459

For more details about this annex : see Official Journal of the European Patent Office, No. 12/82